# FASCICULE DE BREVET EUROPEEN

(11) **EP 1 052 993 B1**
(45) Date de publication et mention de la délivrance du brevet: **02.01.2003**
(21) Numéro de dépôt: 99903744.3
(22) Date de dépôt: 12.02.1999
(51) Int. Cl.: A61K 31/475, A61K 31/535, A61K 31/415, A61K 31/195, A61P 15/00

(54) **COMBINAISON D'UN ANTAGONISTE ALPHA-ADRENERGIQUE ET D'UN AGENT DONNEUR D'OXYDE D'AZOTE POUR LE TRAITEMENT DE DYSFONCTIONS SEXUELLES FEMININES**
ZUSAMMENSETZUNG VON ALPHA-ADRENERGen ANTAGONISTEN MIT STICKSTOFFOXiDDONOREN ZUR BEHANDLUNG VON SEXUALFUNKTIONSSTÖRUNGEN BEI FRAUEN
COMBINATION OF AN ALPHA-ADRENERGIC ANTAGONIST AND A NITROGEN OXIDE DONOR FOR TREATING FEMALE SEXUAL DYSFUNCTION

(30) Priorité: 12.02.1998 FR 9801690; 29.09.1998 FR 9812172
(43) Date de publication de la demande: 22.11.2000
(73) Titulaire: Medical & Pharma Development and Investment Company Limited, Dublin 2 (IE)
(72) Inventeur: GORNY, Philippe, F-75116 Paris (FR)
(74) Mandataire: Tonnellier, Jean-Claude
(86) Numéro de dépôt international: FR9900318
(87) Numéro de publication internationale: WO99040917

(56) Documents cités:
- WO-A-94/04120
- WO-A-95/05172
- WO-A-96/33705
- WO-A-97/27749

## Description

L'invention concerne un médicament destiné à prévenir ou traiter certaines dysfonctions sexuelles féminines.

On sait que, chez les femmes, l'excitation sexuelle se traduit notamment par une vasodilatation des vaisseaux sanguins irriguant les organes génitaux. Cette vasodilatation entraîne en particulier un gonflement du clitoris, ainsi qu'une vasocongestion de la paroi vaginale avec exsudation de fluides vaginaux.

On sait également que, chez les femmes, les réponses physiologiques à la stimulation sexuelle peuvent se trouver altérées temporairement, et parfois durablement, même sans cause organique décelable. Les troubles les plus fréquemment constatés incluent l'absence de désir sexuel même après stimulation, la difficulté d'obtenir un orgasme, la faible intensité du plaisir sexuel et la diminution ou l'absence de la lubrification vaginale naturelle, et ces troubles ont souvent pour conséquence le manque d'intérêt pour l'activité sexuelle. Ce sont ces troubles de la réponse physiologique et/ou anatomique à l'excitation sexuelle que l'on appelle, dans la présente demande, "dysfonctions sexuelles féminines". Selon certaines estimations, une femme sur trois environ souffrirait de telles dysfonctions, temporairement ou de façon chronique.

Il est donc souhaitable de disposer de traitements permettant de réduire la gravité et/ou la durée de ces troubles, ou d'en prévenir l'apparition, et de restaurer la capacité d'accomplissement d'une activité sexuelle satisfaisante, chez les sujets qui présentent de tels troubles, ou qui en redoutent la survenue.

On a proposé à cet effet l'utilisation, principalement chez l'homme, de vasodilatateurs par voie transdermique, transmuqueuse, intra-nasale ou rectale (WO 95/05172) ou par voie orale (WO 96/33705).

Parmi les agents vasodilatateurs, on peut distinguer notamment les agents ayant des effets antagonistes sur les récepteurs α-adrénergiques, qui inhibent le tonus adrénergique et favorisent ainsi la dilatation des artères, et les agents jouant le rôle de donneurs de monoxyde d'azote (NO), soit directement, soit au cours de leur métabolisme. On sait en effet que les cellules endothéliales, qui tapissent la face interne des vaisseaux sanguins, sont capables de sécréter une substance ayant pour effet de dilater les artères, cette substance étant le monoxyde d'azote. Il a été établi que le monoxyde d'azote stimule la synthèse de la guanosine monophosphate cyclique (ou GMPc) qui est l'agent effectif de la relaxation musculaire des artères. On sait aussi que le monoxyde d'azote est le principal neurotransmetteur physiologique mis en jeu par les neurones périphériques non adrénergiques et non cholinergiques.

Parmi les antagonistes des récepteurs α-adrénergiques, encore appelés "α-bloquants", on peut citer notamment la yohimbine, la phentolamine, la tolazoline, la phénoxybenzamine, la chlorpromazine, le piperoxan et la thymoxamine ainsi que, le cas échéant, les sels pharmaceutiquement acceptables de ces composés.

Parmi les produits agissant sur la dilatation des artères par production de monoxyde d'azote, on peut citer par exemple l'arginine, le nitroprussiate de sodium, des nitrates organiques (trinitrate de glycérol, mononitrate ou dinitrate d'isosorbide), des nitrites organiques (nitrites d'amyle ou de butyle), des thionitrites tels que décrits dans le document WO 96/16645 (par exemple S-nitrosocystéine, S-nitrosoglutathion), la molsidomine, ainsi que, le cas échéant, les sels pharmaceutiquement acceptables de ces composés.

Il existe en outre des donneurs de NO qui ont également une activité d'α-bloquants ; ce sont notamment les α-bloquants, substitués par un ou plusieurs groupes nitro (NO₂) ou nitroso (NO), décrits dans le document WO 97/27749. Les α-bloquants qui peuvent être substitués par un ou plusieurs groupes nitro ou nitroso peuvent être choisis parmi pratiquement tous les α-bloquants connus. Ce sont notamment des haloalkylamines comme la phénoxybenzamine ou la dibenamine ; des imidazolines comme la phentolamine, la tolazoline, l'idazoxan, etc. ; des quinazolines comme la prazosine, la terazosine, la doxazosine, etc. ; des dérivés d'indole comme le carvedilol ; des alcools tels que l'ifenprodil ou le labetalol ; des alcaloïdes tels que l'ergocornine, l'ergocristine, l'ergocryptine, la yohimbine, la rauwolscine, etc. ; ou des dérivés de pipéridine comme l'haloperidol.

On a maintenant découvert que l'association de deux activités différentes, l'une étant une activité d'antagoniste des récepteurs α-adrénergiques et l'autre étant une activité de donneur de monoxyde d'azote, permet d'obtenir des résultats favorables dans la prévention et le traitement des troubles de la réponse physiologique et anatomique à la stimulation sexuelle chez la femme, et donc de lutter contre lesdits troubles, grâce à un effet de synergie.

Dans la présente demande, on entend par "donneur de monoxyde d'azote" tout agent qui est capable de produire directement du monoxyde d'azote *in vivo,* tout précurseur métabolique d'un tel agent, ainsi que tout agent susceptible de favoriser la production de monoxyde d'azote endogène.

L'invention peut être mise en oeuvre à l'aide d'un médicament contenant, en association, un agent antagoniste des récepteurs α-adrénergiques et un agent donneur de monoxyde d'azote. Bien entendu, dans le cas où l'on utiliserait un agent ayant ces deux types d'activité, l'association peut se réduire à l'utilisation d'un seul agent.

Les antagonistes des récepteurs α-adrénergiques et les agents donneurs de monoxyde d'azote présents dans ce médicament sont des composés acceptables en pharmacie qui peuvent être choisis notamment parmi ceux qui ont été mentionnés ci-dessus.

On utilise le médicament de l'invention de façon à administrer à la personne traitée des doses efficaces qui peuvent être déterminées à l'aide de tests appropriés. Il convient de remarquer ici que de nombreux α-bloquants et de nombreux donneurs de monoxyde d'azote sont connus, de même que leurs doses actives. En comparant les effets de l'association avec les effets de chacun de ses ingrédients actifs, on constate que l'association permet en général de réduire les doses de l'un au moins des ingrédients actifs. On peut ainsi sélectionner les associations présentant un effet de synergie.

L'invention a également pour objet l'utilisation en association d'un antagoniste des récepteurs α-adrénergiques et d'un agent donneur de monoxyde d'azote comme ingrédients actifs dans la préparation d'un médicament destiné à prévenir ou traiter les troubles de la réponse physiologique et anatomique à la stimulation sexuelle chez la femme. Ce médicament est administré à des sujets qui en ont besoin, c'est-à-dire les personnes ayant éprouvé ou redoutant de tels troubles.

Les ingrédients actifs d'un médicament obtenu selon l'invention peuvent être présentés de façon séparée, chacun sous une forme pharmaceutique appropriée, et réunis dans un même emballage.

Mais pour faciliter l'administration simultanée des ingrédients actifs, on préfère généralement préparer le médicament sous une seule forme pharmaceutique contenant les deux activités associées (y compris sous la forme d'un seul ingrédient actif ayant ces deux activités).

Le médicament de l'invention peut être administré par voie orale, sublinguale, nasale, pulmonaire, rectale, transmuqueuse ou transdermique.

A cet effet, il peut être présenté sous toute forme permettant l'administration par voie orale (en particulier sous la forme de gélules, de solutions ou émulsions buvables, de poudres, de gels, de granulés, de tablettes ou de comprimés), par voie nasale (par exemple sous forme de solutions à administrer sous forme de gouttes ou de pulvérisations), par voie pulmonaire (notamment sous forme de solutions en flacon pressurisé pour aérosols), par voie rectale (suppositoires), par voie cutanée (par exemple onguents ou dispositifs transdermiques, encore appelés timbres ou patches), ou par voie transmuqueuse comme par exemple par voie sublinguale (en particulier sous forme de solutions en flacon pressurisé, ou de comprimés à délitement buccal) ou vaginale (notamment crèmes ou ovules gynécologiques).

Ces formes pharmaceutiques sont préparées de façon usuelle et peuvent contenir des excipients et véhicules classiques appropriés.

Parmi les associations utilisées conformément à l'invention, on citera notamment celle de la yohimbine avec l'arginine.

La yohimbine est une substance extraite de l'écorce de la plante *Corynanthe yohimbe*. Elle a des propriétés d'antagoniste des récepteurs alpha-2-adrénergiques présynaptiques. Autrement dit, elle antagonise le tonus α₂-adrénergique. Elle a été proposée chez l'homme, dans le traitement des impuissances d'origine psychogène et dans certaines formes d'impuissances organiques, notamment en cas de diabète. Divers effets secondaires tels que vertiges, anxiété, nervosité, céphalées, insomnies et augmentation de la tension artérielle ont parfois été observés, mais pour des doses relativement élevées de yohimbine ; voir par exemple The Medical Letter, Edition française, vol.17, n° 2, 5-6 (ML USA n° 938), 1995.

La L-arginine est un précurseur du monoxyde d'azote endogène, et son administration se traduit notamment par un effet sur la relaxation musculaire des artères. L'administration chez l'homme de L-arginine aurait un effet favorable sur les dysfonctions érectiles dans certains cas ; voir A.W. ZORGNIOTTI et E.F. LIZZA, Int. J. Impotence Res., 6, 33-36 (1994).

La yohimbine et/ou l'arginine peuvent être utilisées sous forme non salifiée, ou sous forme salifiée.

Un médicament utilisable selon l'invention peut être préparé sous une forme pharmaceutique permettant l'administration d'une dose suffisante de yohimbine, par exemple de 2 à 10 mg, notamment de 2 à 8 mg, en particulier de 4 à 6 mg, en une ou deux prises. Cette dose est calculée en poids de yohimbine sous forme de base libre. Le médicament est préparé sous une forme pharmaceutique permettant en outre l'administration d'une dose suffisante d'arginine qui est par exemple une dose de 1 à 8 g par jour, notamment de 2 à 6 g, en une ou deux prises, ladite dose étant calculée en poids d'arginine sous forme de base libre.

Par exemple, on peut administrer une dose de 2 à 6 mg de yohimbine et de 1 à 4 g par jour de L-arginine, chez l'adulte, pour un traitement devant durer de 2 à 4 semaines. Dans le cas d'une utilisation ponctuelle, on peut administrer, par exemple, de 4 à 10 mg, en particulier de 4 à 6 mg de yohimbine, et de 2 à 6 g de L-arginine en une seule prise, environ 1 à 2 heures avant un rapport sexuel envisagé.

Les exemples suivants illustrent l'invention.

### EXEMPLES

### EXEMPLE 1 : Gélules

On prépare une gélule constituée par une capsule de gélatine contenant :
- Arginine : 0,5 g
- Yohimbine : 1 mg

La yohimbine peut être utilisée sous forme de base libre ou sous forme d'un sel tel que le chlorhydrate.

L'arginine peut être utilisée sous la forme de base libre ou d'un sel acceptable en pharmacie, tel que le chlorhydrate, le glutamate, l'aspartate ou le citrate.

Dans le cas de troubles chroniques, on peut envisager une administration quotidienne de façon systématique. La durée du traitement peut varier par exemple de 2 à 4 semaines ou davantage.

On peut ensuite préconiser une utilisation épisodique, à la demande.

De façon analogue, on prépare des gélules contenant, soit :
- Yohimbine : 2 mg
- Arginine : 0,5 g
soit :
- Phentolamine mésylate : 20 mg
- Molsidomine : 2 mg

### EXEMPLE 2 : Sachets de poudre pour suspension buvable

On prépare des sachets de poudre contenant :
- Chlorhydrate de yohimbine : 6 mg
- Glutamate d'arginine : 6 g
- Excipient aromatisé : 3 g

### EXEMPLE 3 : Essais

Des essais ont été effectués sur dix femmes volontaires, âgées de 27 à 60 ans, vivant en couple, et souffrant de l'un au moins des troubles suivants : perte du désir sexuel, impossibilité d'obtenir un orgasme, diminution de l'intensité du plaisir sexuel, ou sécheresse vaginale. On a retenu pour ce test des femmes dont les partenaires ne souffraient pas eux-mêmes de dysfonctions sexuelles.

On a remis aux personnes testées des sachets contenant 6 g de glutamate d'arginine sous forme de poudre à dissoudre dans l'eau, ainsi que des comprimés dosés à 2 mg de yohimbine, en leur demandant d'ingérer simultanément le contenu d'un sachet de poudre ainsi que 3 comprimés, 1 à 2 heures avant une activité sexuelle envisagée, et de respecter des intervalles d'au moins 24 heures entre les prises.

Les personnes testées ont évalué subjectivement les effets observés globalement, après 5 prises, sur les points suivants : désir sexuel, obtention d'un orgasme, intensité du plaisir sexuel et lubrification vaginale.

6 des personnes testées ont constaté une amélioration sur au moins un des critères retenus dans cette étude. Les 4 autres n'ont constaté aucune amélioration.

Parallèlement, des essais similaires ont été effectués sur 3 femmes ne souffrant d'aucune dysfonction sexuelle. Ces 3 femmes ont constaté une augmentation de l'intensité et de la durée des orgasmes.

## Revendications

1. Utilisation, en association, d'un agent ayant une activité d'antagoniste des récepteurs α-adrénergiques et d'un agent ayant une activité de donneur de monoxyde d'azote comme ingrédients actifs dans la préparation d'un médicament destiné à prévenir ou traiter les troubles de la réponse physiologique et anatomique à la stimulation sexuelle chez la femme.

2. Utilisation selon la revendication 1, dans laquelle ledit agent antagoniste des récepteurs α-adrénergiques est choisi parmi la yohimbine, la phentolamine, la tolazoline, la phénoxybenzamine, la chlorpromazine, le piperoxan et la thymoxamine, et leurs sels pharmaceutiquement acceptables.

3. Utilisation selon l'une quelconque des revendications précédentes, dans laquelle l'agent donneur de monoxyde d'azote est un composé choisi parmi l'arginine, le nitroprussiate de sodium, des nitrates ou nitrites organiques, des thionitrites et la molsidomine, ainsi que, le cas échéant, les sels pharmaceutiquement acceptables de ces composés.

4. Utilisation selon l'une quelconque des revendications précédentes, dans laquelle l'agent antagoniste des récepteurs α-adrénergiques est la yohimbine.

5. Utilisation selon la revendication précédente, dans laquelle on prépare ledit médicament sous une forme pharmaceutique permettant l'administration d'un dose de 2 à 10 mg, notamment de 2 à 8 mg de yohimbine, et en particulier de 4 à 6 mg, en une ou deux prises, ladite dose étant calculée en poids de yohimbine sous forme de base libre.

6. Utilisation selon l'une quelconque des revendications précédentes, dans laquelle l'agent donneur de monoxyde d'azote est l'arginine.

7. Utilisation selon la revendication précédente, dans laquelle on prépare ledit médicament sous une forme pharmaceutique permettant l'administration d'une dose de 1 à 8 g d'arginine, et en particulier de 2 à 6 g, en une ou deux prises, ladite dose étant calculée en poids d'arginine sous forme de base libre.

8. Utilisation selon l'une quelconque des revendications précédentes, dans laquelle l'antagoniste des récepteurs α-adrénergiques est la yohimbine sous forme libre ou salifiée, et l'agent donneur de monoxyde d'azote est l'arginine, sous forme libre ou salifiée.

9. Utilisation selon la revendication 1, dans laquelle ledit médicament contient comme ingrédient actif au moins un α-bloquant, substitué par un ou plusieurs groupements nitro ou nitroso, ayant à la fois une activité antagoniste des récepteurs α-adrénergiques et une activité de donneur de monoxyde d'azote.

10. Utilisation selon l'une quelconque des revendications précédentes, dans laquelle ledit médicament présente l'une au moins des caractéristiques suivantes :
- il contient lesdits ingrédients actifs, de façon séparée, dans un même emballage ;
- il se présente sous une forme pharmaceutique unique contenant les deux ingrédients actifs ;
- il se présente sous une forme pharmaceutique unique contenant un ingrédient actif tel que défini dans la revendication 9 ;
- il se présente sous la forme de gélules, de solutions ou émulsions buvables, de granulés, de gels, de poudres, de tablettes, de comprimés, d'onguents, de dispositifs transdermiques, de suppositoires, de crèmes, d'ovules gynécologiques ou encore sous la forme de solutions, éventuellement en flacons pressurisés, pour administration par voie nasale ou pulmonaire.

## Patentansprüche

1. Verwendung eines Mittels mit einer antagonistischen Wirkung für α-adrenerge Rezeptoren und eines Mittels mit einer Stickstoffmonoxid-Donoraktivität in Assoziation als aktive Bestandteile bei der Herstellung eines Medikaments zur Vorbeugung oder Behandlung von Störungen bzw. Problemen der physiologischen und anatomischen Antwort auf sexuelle Stimulation bei der Frau.

2. Verwendung gemäß Anspruch 1, worin der Antagonist der α-adrenergen Rezeptoren ausgewählt ist unter Yohimbin, Phentolamin, Tolazolin, Phenoxybenzamin, Chlorpromazin, Piperoxan und Thymoxamin sowie deren pharmazeutisch annehmbaren Salzen.

3. Verwendung gemäß einem der vorstehenden Ansprüche, worin der Stickstoffmonoxid-Donor eine Verbindung ist, ausgewählt unter Arginin, Natriumnitroprussiat, organischen Nitraten oder Nitriten, Thionitriten und dem Molsidomin wie auch gegebenenfalls den pharmazeutischen annehmbaren Salzen dieser Verbindungen.

4. Verwendung nach einem der vorstehenden Ansprüche, worin der Antagonist der α-adrenergen Rezeptoren das Yohimbin ist.

5. Verwendung nach dem vorstehenden Anspruch, worin man das Medikament in einer pharmazeutischen Form herstellt, die die Verabreichung einer Dosis von 2 bis 10 mg, insbesondere 2 bis 8 mg Yohimbin und insbesonders von 4 bis 6 mg in ein oder zwei Chargen gestattet, wobei die Dosis in Gewicht des Yohimbins in Form der freien Base berechnet wird.

6. Verwendung nach einem der vorstehenden Ansprüche, worin der Stickstoffmonoxid-Donor das Arginin ist.

7. Verwendung gemäß dem vorstehenden Anspruch, worin man das Medikament in einer pharmazeutischen Form herstellt, welche die Verabreichung einer Dosis von 1 bis 8 g Arginin und insbesondere 2 bis 6 g in einer oder zwei Chargen gestattet, wobei die Dosis in Gewicht des Arginins in Form der freien Base berechnet ist.

8. Verwendung nach einem der vorstehenden Ansprüche, worin der Antagonist der α-andrenergen Rezeptoren das Yohimbin in freier oder versalzter Form ist ist und der Stickstoffmonoxid-Donor das Arginin in freier oder versalzter Form ist.

9. Verwendung gemäß Anspruch 1, worin das Medikament als aktivem Bestandteil mindestens einen α-Blocker, substituiert mit einer oder mehreren Nitro- oder Nitrosogruppen, mit gleichzeitig einer antagonistischen Aktivität für α-andrenerge Rezeptoren und einer Stickstoffmonoxid-Donoraktivität enthält.

10. Verwendung nach einem der vorstehenden Ansprüche, worin das Medikament mindestens eine der folgenden Eigenschaften aufweist:
- es enthält die aktiven Bestandteile in getrennter Form in ein und derselben Verpackung;
- es liegt in einer einzigen pharmazeutischen Form vor, enthaltend die beiden aktiven Bestandteile;
- es liegt in einer einzigen pharmazeutischen Form vor, enthaltend einen aktiven Bestandteil wie in Anspruch 9 definiert;
- es liegt in Form von Dragees, Trinklösungen oder Trinkemulsionen, von Granulat, Gelen, Pulvern, Tabletten, Presslingen, Salben, transtermalen Verbänden, Zäpfchen, Cremes, von gynäkologischen Ovula oder auch in Form von Lösungen für die Verabreichung auf nasalem oder pulmonarem Wege, gegebenenfalls in Druckbehältern, vor.

## Claims

1. Use, in combination, of an agent having α-adrenergic receptor antagonist activity and of an agent having nitrogen monoxide donor activity, as active ingredients in the preparation of a medicinal product for preventing or treating disorders in the physiological and anatomical response to sexual stimulation in women.

2. Use according to Claim 1, in which the said α-adrenergic receptor antagonist is chosen from yohimbine, phentolamine, tolazoline, phenoxybenzamine, chlorpromazine, piperoxan and thymoxamine, and the pharmaceutically acceptable salts thereof.

3. Use according to either of the preceding claims, in which the nitrogen monoxide donor is a compound chosen from arginine, sodium nitroprusside, organic nitrates or nitrites, thionitrites and molsidomine, as well as, where appropriate, the pharmaceutically acceptable salts of these compounds.

4. Use according to any one of the preceding claims, in which the α-adrenergic receptor antagonist is yohimbine.

5. Use according to the preceding claim, in which the said medicinal product is prepared in a pharmaceutical form allowing the administration of a dose of from 2 mg to 10 mg, especially from 2 mg to 8 mg and in particular from 4 mg to 6 mg, of yohimbine, in one or two dosage intakes, the said dose being calculated as the weight of yohimbine in free base form.

6. Use according to any one of the preceding claims, in which the nitrogen monoxide donor is arginine.

7. Use according to the preceding claim, in which the said medicinal product is prepared in a pharmaceutical form allowing the administration of a dose of from 1 g to 8 g and in particular from 2 g to 6 g of arginine, in one or two dosage intakes, the said dose being calculated as the weight of arginine in free base form.

8. Use according to any one of the preceding claims, in which the α-adrenergic receptor antagonist is yohimbine in free or salified form, and the nitrogen monoxide donor is arginine, in free or salified form.

9. Use according to Claim 1, in which the said medicinal product contains, as active ingredient, at least one α-blocker, substituted with one or more nitro or nitroso groups, having both α-adrenergic receptor antagonist activity and nitrogen monoxide donor activity.

10. Use according to any one of the preceding claims, in which the said medicinal product has at least one of the following characteristics:
- it contains the said active ingredients separately in the same packaging;
- it is in a single pharmaceutical form containing the two active ingredients;
- it is in a single pharmaceutical form containing an active ingredient as defined in Claim 9;
- it is in the form of gel capsules, drinkable solutions or emulsions, granules, gels, powders, lozenges, tablets, ointments, transdermal devices, suppositories, creams or gynaecological pessaries, or alternatively in the form of solutions, optionally in pressurized bottles for nasal or pulmonary administration.
